(19) Europäisches Patentamt
European Patent Office
Office européen des brevets

(11) Veröffentlichungsnummer : **0 354 444 B1**

(12) # EUROPÄISCHE PATENTSCHRIFT

(45) Veröffentlichungstag der Patentschrift : **20.01.93 Patentblatt 93/03**

(51) Int. Cl.$^5$ : **C07C 17/20,** C07B 39/00, C07C 255/50, C07C 205/12

(21) Anmeldenummer : **89114161.6**

(22) Anmeldetag : **01.08.89**

(54) **Verfahren zum Einführen von Fluoratomen an aromatische Kerne durch nucleophilen Austausch.**

(30) Priorität : **12.08.88 DE 3827436**

(43) Veröffentlichungstag der Anmeldung : **14.02.90 Patentblatt 90/07**

(45) Bekanntmachung des Hinweises auf die Patenterteilung : **20.01.93 Patentblatt 93/03**

(84) Benannte Vertragsstaaten : **BE DE FR GB IT**

(56) Entgegenhaltungen :
**EP-A- 0 003 344**
**WO-A-87/04151**
**DE-A- 2 938 939**
**GB-A- 2 039 473**
**US-A- 3 992 432**

(56) Entgegenhaltungen :
**PATENT ABSTRACTS OF JAPAN, Band 7, Nr. 46 (C-153)(1191), 23. Februar 1983; & JP-A-57 197 226**
**PATENT ABSTRACTS OF JAPAN, Band 9, Nr. 287 (C-314)(2010), 14. November 1985; & JP-A-60 130 537**
**PATENT ABSTRACTS OF JAPAN, Band 10, Nr. 230 (C-365)(2286), 9. August 1986; & JP-A-61 65 830**

(73) Patentinhaber : **BAYER AG**
**W-5090 Leverkusen 1 Bayerwerk (DE)**

(72) Erfinder : **Kysela, Ernst, Dr.**
**Virchowstrasse 14**
**W-5060 Bergisch Gladbach 1 (DE)**
Erfinder : **Braden, Rudolf, Dr.**
**Nothauser Feld 1**
**W-5068 Odenthal (DE)**



Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein besonderes vorteilhaftes Verfahren zum Einführen von Fluoratomen an aromatische Kerne durch nucleophilen Austausch, insbesondere durch Austausch von Halogenatomen und/oder Nitrogruppen.

Es sind eine Reihe von Verfahren bekannt, mit denen es gelingt, an aromatischen Kernen befindliche Atomgruppen oder Atome, z.B. Halogenatome und/oder Nitrogruppen, gegen Fluoratome auszutauschen. Bekannte Verfahren wenden als Fluorierungsmittel Kaliumfluorid an, das im allgemeinen in Gegenwart eines hochsiedenden, inerten Lösungsmittels und gegebenenfalls in Gegenwart von Phasentransferkatalysatoren eingesetzt wird (s. z.B. DE-OS 2 938 939 oder EP-OS 0 003 344). Geeignete Phasentransferkatalysatoren sind beispielsweise quarternäre Ammoniumsalze, quaternäre Phosphoniumsalze und Kronenether. Nachteilig sind die immer noch anzuwendenden relativ hohen Temperaturen, was negative Einflüsse auf den Energiebedarf, die Korrosion, die Möglichkeit von Zersetzungsreaktionen und die Lebenszeit der Phasentransferkatalysatoren hat.

Aus Patent Abstracts of Japan Vol. 7 (1983) No. 46 (C153) [1191] ist die Umsetzung von Chlor- oder Bromaromaten mit Kaliumfluorid und einem weiteren Alkalioder Erdalkalifluorid in Gegenwart von Kronenethern bekannt. Auch hier ist das Verhältnis von Reaktionstemperatur zu Reaktionszeit (bis 20 Stunden) ungünstig.

Es wurde nun ein Verfahren zur Herstellung von aromatischen, kernfluorierten Verbindungen durch nucleophilen Austausch mit Kaliumfluorid in Gegenwart von Phasentransferkatalysatoren, weiteren Metallsalzen und gegebenenfalls Lösungsmitteln gefunden, das dadurch gekennzeichnet ist, daß man die Umsetzung in zusätzlicher Gegenwart von Salzen von Metallen der 3. bis 5. Hauptgruppe des Periodensystems der Elemente und von Nebengruppenelementen durchführt. Bevorzugt sind Halogenide und Sulfate, besonders bevorzugt Chloride. Beispielsweise kommen infrage: Chromsalze wie $CrCl_3$ x $6H_2O$, Eisensalze wie $FeCl_3$, Kobaltsalze wie $CoCl_2$ x $6H_2O$, Nickelsalze wie $NiCl_2$ x $6H_2O$, Kupfersalze wie $CuSO_4$, Aluminiumsalze wie $AlCl_3$, Zinksalze wie $ZnCl_2$ und Antimonsalze wie $SbCl_3$.

Die Metallsalze können in unterschiedlichen Mengenverhältnissen eingesetzt werden. Bezogen auf ein Mol eingesetzten Phasentransferkatalysator kann man beispielsweise 0,1 bis 10 Mole Metallsalz einsetzen. Bevorzugt beträgt diese Menge 0,2 bis 5 Mole, insbesondere 0,25 bis 1 Mol.

Während der Fluorierungsreaktion können der jeweils eingesetzte Phasentransferkatalysator und das jeweils eingesetzte Metallsalze ganz oder teilweise als in situ gebildete Komplexe vorliegen, z.B. als Komplexe des Typs

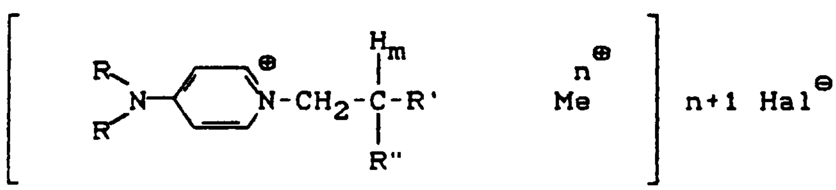

wobei

R unabhängig voneinander jeweils einen einbindingen organischen Rest bedeutet, z.B. jeweils $C_1$- bis $C_{10}$-Alkyl, und

R′ und R″ jeweils eine $C_1$- bis $C_{10}$-Alkylgruppe oder gemeinsam mit dem dazwischen befindlichen C-Atom einen gesättigten carbocyclischen Ring mit insgesamt 5 bis 6 C-Atomen bedeutet, wobei dann m jeweils für 1 steht oder wobei R′ und R″ gemeinsam mit dem dazwischen befindlichen C-Atom einen carbocyclischen aromatischen Ring mit 6 C-Atomen bedeutet, wobei dann m für Null steht,

Me ein Metallion mit n positiven Ladungen und

Hal Halogen bedeutet.

Der jeweils eingesetzte Phasentransferkatalysator und/oder das jeweils eingesetzte Metallsalz können auch ganz oder teilweise in Form von Komplexen in das Reaktionsgemisch eingebracht werden.

Als Phasentransferkatalysatoren kommen beispielsweise quartäre Stickstoffverbindungen, quartäre Phosphorverbindungen, Pyridiniumsalze und Kronenether infrage. Die 3 erstgenannten Katalysatortypen können beispielsweise den Formeln

$$[NR_4]^{\oplus} Hal^{\ominus}, [PR_4]^{\oplus} Hal^{\ominus} \text{ oder}$$

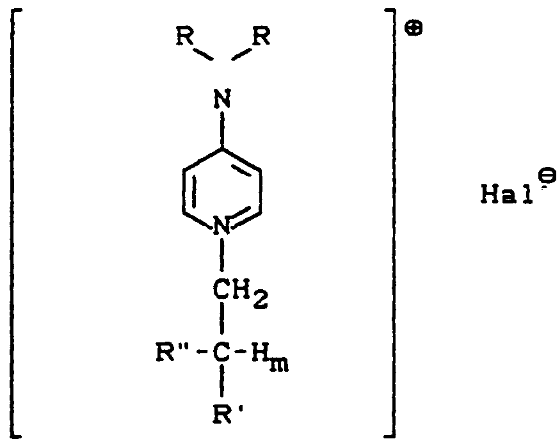

wobei

R, R′, R″, m und Hal die oben angegebene Bedeutung haben,

entsprechen.

Vorzugsweise verwendet man quarternäre Stickstoffverbindungen oder Pyridiniumsalze, beispielsweise Methyl-trioctyl-ammoniumchlorid, Tetrabutyl-ammoniumchlorid, Tetrabutyl-ammoniumbromid,Tetraethylammoniumchlorid, Triethyl-benzyl-ammoniumchlorid, Dimethyl-benzyl-phenyl-ammoniumchlorid, Dimethyldodecyl-benzylammoniumchlorid, Tetraphenyl-phosphoniumbromid, Triphenyl-benzyl-phosphoniumbromid, N′,N′-Dimethylamino-N-benzyl-pyridiniumchlorid, N′,N′-Dimethylamino-N-2-dimethylpropyl-pyridiniumchlorid. Beim Einsatz von Kronenethern ist der Ether 18 Krone 6 bevorzugt.

Die Phasentransferkatalysatoren können z.B. in Mengen von 0,1 bis 20 Mol-%, bezogen auf das Edukt eingesetzt werden. Vorzugsweise beträgt diese Menge 0,5 bis 5 Mol-%.

Das erfindungsgemäße Verfahren muß nicht immer in Gegenwart von Lösungsmitteln durchgeführt werden. Anstelle von Lösungsmitteln kann man auch einen Überschuß der zu fluorierenden aromatischen Verbindung einsetzen. Häufig erhält man beim Einsatz von Nitroaromaten auch ohne Lösungsmittel gute Ergebnisse. Trotzdem ist es im allgemeinen bevorzugt, Lösungsmittel zu verwenden.

Brauchbare Lösungsmittel sind praktisch alle aprotischen, dipolaren Lösungsmittel, die einen ausreichend hohen Siedepunkt haben und unter den Reaktionsbedingungen ausreichend inert sind, beispielsweise Dimethylsulfoxid, N-Methylpyrrolidon, Tetramethylensulfon, Benzonitril, Nitrobenzol, Dimethylacetamid, Ethylenglykoldimethylether und Diglyme.

Die Reaktionstemperaturen für das erfindungsgemäße Verfahren können z.B. im Bereich 100 bis 250°C liegen. Vorzugsweise arbeitet man im Bereich 130 bis 180°C.

Das Kaliumfluorid kann in verschiedenen Mengen eingesetzt werden. Im allgemeinen setzt man die stöchiometrisch erforderliche Menge oder mehr ein. Große Überschüsse sind nicht kritisch, jedoch wirtschaftlich nicht sinnvoll. Vorzugsweise setzt man deshalb pro Mol einzuführender Fluoratome 1,1 bis 2 Mole Kaliumfluorid ein.

Als Edukt können in das erfindungsgemäße Verfahren Aromaten mit verschiedenenartigen Abgangsgruppen eingesetzt werden. Vorzugsweise sind die zum Austausch gegen Fluor vorgesehenen Abgangsgruppen durch einen oder mehrere weitere -I und/oder -M-Substituenten aktiviert, beispielsweise durch Halogen, CN, CO-Halogen, $SO_2$-Halogen oder $CF_3$. Bevorzugte Edukte enthalten als Abgangsgruppen Halogen- und/oder Nitrosubstituenten, beispielsweise 1 bis 3 an den aromatischen Kern gebundene Chloratome und/oder 1 bis 2 an den aromatischen Kern gebundene Nitrogruppen. Als Beispiele für Edukte seien genannt:

3,4-Dichlor-nitrobenzol, 2,4-Dichlor-nitrobenzol, 2,4-Dichlor-5-fluor-benzoylchlorid, 3,4-Dichlor-benzoylchlorid, 2,6-Dichlor-benzonitril, 2-Nitro-6-chlorbenzonitril, 2-Trifluormethyl-4-nitro-chlorbenzol, Pentachlorbenzotrifluorid, 4-Trifluormethyltetrachlor-benzoylchlorid und 3,4,5-Trichlor-benzotrifluorid.

Vorzugsweise werden mit dem erfindungsgemäßen Verfahren am aromatischen Kern befindliche Halogenatome und/oder Nitrogruppen gegen Fluoratome ausgetauscht. Insbesondere kann man auch mehrere derartige Halogenatome und/oder Nitrogruppen, vorzugweise bis zu 5 Chloratome und/oder 1 bis 2 Nitrogruppen gegen Fluor austauschen.

Als Gefäßmaterialien für die Durchführung der erfindungsgemäßen Reaktion kommen beispielsweise Glas, mit Nickel und/oder Chrom legierte Stähle und perfluorierte Olefinpolymere infrage. Perfluorierte Olefinpolymere, z.B. Polytetrafluorethylen, sind bevorzugt. Bei Verwendung von Metallgefäßen kann nicht ausgeschlossen werden, daß in geringem Umfang aus Gefäßmaterialien stammende Metallsalze in das Reaktionsgemisch gelangen. Dieser Effekt kann unter bestimmten Umständen (z.B. bei brauchbarer Art und Menge des gebildeten Metallsalzes) erwünscht sein.

Alle Komponenten für das Reaktionsgemisch kommen vorzugsweise in trockener bzw. weitgehend wasserfreier Form zum Einsatz. Gegebenenfalls kann man im Reaktionsgemisch vorliegende geringe Wassermengen durch Abdestillieren (gegebenenfalls zusammen mit einem geringen Anteil des verwendeten Lösungsmittels) oder durch azeotrope Destillation mit einem Schleppmittel entfernen.

Das nach der Reaktion vorliegende Gemisch kann je nach den darin vorliegenden Verhältnissen auf unterschiedliche Weise aufgearbeitet werden.

Bei Gegenwart eines Lösungmsittels, das höher siedet als das aromatische, kernfluorierte Produkt, kann man destillieren, bis das Lösungsmittel übergeht und gegebenenfalls aus dem noch vorliegenden salzhaltigen Rückstand das Lösungsmittel (weitgehend) durch Destillieren oder Filtrieren zurückgewinnen. Bei Gegenwart eines Lösungsmittels, das niedriger siedet als das aromatische, kernfluorierte Produkt, kann man zunächst durch Destillation das Lösungsmittel entfernen und dann das Produkt durch weitere Destillation oder Extraktion gewinnen. Bei Gegenwart eines Lösungsmittels, das durch Destillation nur schwierig vom aromatischen kernfluorierten Produkt abzutrennen ist, kann man das Produkt entweder durch Extraktion oder durch Einbringen des gegebenenfalls filtrierten Reaktionsgemisches in Wasser und Abtrennen der organischen Phase gewinnen. Bei Abwesenheit eines Fremdlösungsmittels kann man aromatisches, kernfluoriertes Produkt und unumgesetzte Ausgangsverbindung durch Destillation voneinander trennen. Alle angegebenen Destillationen werden vorzugsweise im Vakuum durchgeführt.

Das erfindungsgemäße Verfahren hat den überraschenden Vorteil, daß es bei gegebener Reaktionszeit bei niedrigerer Temperatur oder bei gegebener Temperatur in kürzerer Zeit durchgeführt werden kann. Das bedeutet gegenüber bekannten Verfahren eine Verringerung des Energiebedarfs, der Korrosion und der Möglichkeit von Zersetzungsreaktionen und eine Verlängerung der Lebenszeit der Phasentransferkatalysatoren.

Welche Kombinationen eines Metallsalzes mit einem Phasentransferkatalysator und gegebenenfalls einem Lösungsmittel optimale Ergebnisse liefert, kann gegebenenfalls durch einfache Reihenversuche ermittelt werden. Für unterschiedliche zu fluorierende aromatische Chlor- und/oder Nitroverbindungen kann jeweils eine andere qualitative und/oder quantitative Kombination von Metallsalz, Phasentransferkatalysator und gegebenenfalls Lösungsmittel ein optimales Ergebnis erbringen.

## Beispiel 1

Eine in einem Reaktionsgefäß aus Glas befindliche Mischung aus 87 g (1,5 Mol) Kaliumfluorid und 250 g Tetramethylensulfon wurde zur Wasserentfernung andestilliert bis 25 g Tetramethylensulfon übergangen waren. Danach wurden 192 g 3,4-Dichlor-nitrobenzol, 10 g (0,025 Mol) Methyl(-trioctyl-ammoniumchlorid und 1,65 g (0,0125 Mol) $AlCl_3$ zugesetzt und das Gemisch auf 170°C erwärmt. Nach 4 Stunden betrug der Umsatz 97 % (gaschromatographisch bestimmt). Das Reaktionsgemisch wurde dann durch Destillation aufgearbeitet. Mit einem Siedepunkt von 92-93°C bei 10 mbar wurden 149 g 3-Chlor-4- fluor-nitrobenzol erhalten. Das entspricht einer Ausbeute von 85 % der Theorie.

## Beispiele 2 bis 16

Es wurde verfahren wie in Beispiel 1, jedoch wurden die Art des Metallsalzes, die Art des Phasentransferkatalysators und die Art des Lösungsmittels variiert, und zum Vergleich auch Beispiele ohne Metallsalzzusatz und ohne Zusatz eines Phasentransferkatalysators durchgeführt. Wasserhaltige Metallsalze wurden vor dem Andestillieren in die Mischung aus Kaliumfluorid und Tetramethylensulfon gegeben, um das eingeschleppte Wasser vor Beginn der Umsetzung zu entfernen.

Die Details sind aus Tabelle 1 ersichtlich. In Tabelle 1 steht in der Spalte "Phasentransferkatalystor" A für Methyl-trioctyl-ammoniumchlorid, B für Tetraethylammoniumchlorid, C für Tetrabutylammoniumbromid, D für N′,N′-Dimethylamino-N-2-ethyl-hexyl-pyridiniumchlorid und E für N′,N′-Dimethylamino-N-benzyl-pyridiniumchlorid, in der Spalte "Lösungsmittel" steht TMS für Tetramethylensulfon und in Spalte "Ausbeute..." bedeutet n.a. = nicht aufgearbeitet.

Wurde kein Lösungsmittel eingesetzt, so wurde zur Aufarbeitung mit 50 ml Methylenchlorid verdünnt, filtriert, der feste Rückstand mit 25 ml Methylenchlorid gewaschen und die vereinigten Filtrate destilliert.

**Tabelle 1:**

| Beispiel Nr. | Metallsalz | Phasentransferkatalysator | Lösungsmittel | nach x Stunden erreichter Umsatz von y % x | y | Ausbeute an 3-Chlor-4-fluor-nitro-benzol [% d. Th.] | Bemerkungen |
|---|---|---|---|---|---|---|---|
| 2 | $CrCl_3 \times 6\ H_2O$ | A | TMS | 6 | 97 | 86 | |
| 3 | $CoCl_2 \times 6\ H_2O$ | A | TMS | 6 | 97 | 85 | |
| 4 | $FeCl_3$ | A | TMS | 6 | 97 | 82 | |
| 5 | $CrF_3 \times 4\ H_2O$ | A | TMS | 8 | 97 | 80 | |
| 6 | - | A | TMS | 10 | 97 | 81 | zum Vergleich |
| 7 | $FeCl_3$ | - | TMS | 10 | 46 | n.a. | zum Vergleich |
| 8 | $AlCl_3$ | B | TMS | 6 | 98 | 85 | |
| 9 | - | B | TMS | 10 | 94 | 81 | zum Vergleich |
| 10 | $FeCl_3$ | C | TMS | 6 | 98 | 83 | |
| 11 | - | C | TMS | 10 | 96 | 80 | zum Vergleich |
| 12 | $CrCl_3 \times 6\ H_2O$ | D | - | 4 | 99 | 87 | |
| 13 | $FeCl_3$ | D | - | 4 | 98 | 85 | |
| 14 | - | D | - | 12 | 96 | 87 | zum Vergleich |
| 15 | $FeCl_3$ | E | - | 4 | 95 | 81 | |
| 16 | - | E | - | 8 | 90 | n.a. | zum Vergleich |

Beispiele 17 bis 20

Es wurde verfahren wie in Beispiel 1, jedoch wurde statt 3,4-Dichlor-nitrobenzol eine entsprechende Menge 4-Chlor-nitrobenzol eingesetzt, anstatt Methyl-trioctyl-ammoniumchlorid wurde eine entsprechende Menge N′,N′-Dimethylamino-N-2-ethyl-hexyl-pyridiniumchlorid eingesetzt und anstatt 3-Chlor-4-fluor-nitrobenzol 4-Fluor-nitrobenzol erhalten. Außerdem wurde der Phasentransferkatalysator variiert (Beispiel 19) und Beispiele ohne Metallsalzzusatz zum Vergleich durchgeführt (Beispiele 18 und 20).

Details sind aus Tabelle 2 ersichtlich, in der die gleichen Abkürzungen wie in Tabelle 1 verwendet werden.

**Tabelle 2:**

| Beispiel Nr. | Metallsalz | Phasentransferkatalysator | Lösungsmittel | nach x Stunden erreichter Umsatz von y % x | y | Ausbeute an 4-Fluornitrobenzol [% d. Th.] | Bemerkungen |
|---|---|---|---|---|---|---|---|
| 17 | $CrCl_3$ x 6 $H_2O$ | D | TMS | 6 | 97 | 92 | |
| 18 | - | D | TMS | 12 | 93 | 91 | zum Vergleich |
| 19 | $CrCl_3$ x 6 $H_2O$ | E | TMS | 8 | 99 | 85 | |
| 20 | - | E | TMS | 12 | 97 | 83 | zum Vergleich |

Beispiele 21 bis 23

192 g (1 Mol) 2,4-Dichlornitrobenzol und 87 g (1,5 Mol) Kaliumfluorid wurden unter Zusatz von 0,025 Mol der jeweils angegebenen Verbindung 8 Stunden auf 180°C erwärmt. Anschließend wurde gaschromatographisch der Umsatz bestimmt und nach destillativer Aufarbeitung die Zusammensetzung des Rohdestillats.

Die Details sind aus Tabelle 3 ersichtlich. Die Bedeutung von E ist wie bei der Erläuterung der Tabelle 1 angegeben, E' bedeutet das entsprechende Pyridinium-Kation.

**Tabelle 3:**

| Beispiel Nr. | eingesetzte Verbindung | nach 8 Stunden a = Gew.-% Ausgangsverbindung b = Gew.-% Monofluorverbindung c = Gew.-% Difluorverbindung | | Ausbeute an Difluorverbindung [% d. Th.] |
|---|---|---|---|---|
| 21+) | E | a | 34,6 | 12 |
| | | b | 45,2 | |
| | | c | 16,9 | |
| 22 | $[E'_2Co^{2+}]\ Cl_4^-$ | a | 21,4 | 25 |
| | | b | 49,0 | |
| | | c | 30,0 | |
| 23 | $[E'Fe^{3+}]Cl_4^-$ | a | 22,2 | 24 |
| | | b | 48,0 | |
| | | c | 29,0 | |

+) zum Vergleich

Beispiele 24 bis 28

157 g (1 Mol) 4-Chlor-nitrobenzol, 87 g (1,5 Mol) Kaliumfluorid, 150 g Tetramethylensulfon, 6,77 g (0,025 Mol) N,N'-Dimethylamino-N-2-ethyl-hexylpyridiniumchlorid (= D) und wechselnde Mengen $CrCl_3$ x 6 $H_2O$ wurden zusammengegeben und 16 Stunden auf 170°C erhitzt. Danach wurde gaschromatographisch der Umsatz bestimmt. Die Details sind aus der Tabelle 4 ersichtlich.

Tabelle 4:

| Beispiel Nr. | Molverhältnis D : $CrCl_3$ x 6 $H_2O$ | Umsatz nach 16 Stunden [%] |
|---|---|---|
| 24 | 1 : 0,1 | 84 |
| 25 | 1 : 0,5 | 98 (bereits nach 12 Stunden) |
| 26 | 1 : 1 | 98 |
| 27 | 1 : 2 | 97 |
| 28 | 1 : 2 | 90,7 |

Beispiele 29 bis 33

172 g (1 Mol) 2,6-Dichlorbenzonitril, 174 g (3 Mol) Kaliumfluorid, 0,025 Mol Phasentransferkatalysator (PTC) und 0,0125 Mol Metallsalz wurden zusammen mit 450 g Lösungsmittel 4 Stunden auf 170°C erhitzt. Danach wurde nach destillativer Aufarbeitung die Zusammensetzung des Rohdestillats gaschromatographisch bestimmt. Die Details sind aus Tabelle 5 ersichtlich. In der Spalte "Lösungsmittel" steht TMS für Tetramethylensulfon, BN für Benzonitril und NB für Nitrobenzol. In der Spalte PTC haben die verwendeten Abkürzungen die bei der Erläuterung der Tabelle 1 angegebene Bedeutung.

**Tabelle 5:**

| Beispiel Nr. | Lösungsmittel | PTC | Metallsalz | Gehalt nach 4 h<br>a = Ausgangsprodukt<br>b = Monofluorverbindung<br>c = Difluorverbindung<br>[Gew.-%] |
|---|---|---|---|---|
| 29 | TMS | E | $CrCl_3 \times 6\ H_2O$ | a 28<br>b 48<br>c 23 |
| 30+) | BN | E | - | a 47<br>b 48 |
| 31 | BN | E | $CrCl_3 \times 6\ H_2O$ | a 13<br>b 49<br>c 33 |
| 32+) | NB | D | - | a 72<br>b 20<br>c 7 |
| 33 | NB | D | $FeCl_3$ | a 30<br>b 58<br>c 12 |

+) zum Vergleich

Beispiele 34 und 35

172 g (1 Mol) 2,6-Dichlor-benzonitril, 174 g (3 Mol) Kaliumfluorid, 450 g Dimethylsulfoxid und 0,025 Mol Tetraphenylphosphoniumbromid wurden einmal ohne Gegenwart von $FeCl_3$ (Beispiel 34) und einmal in Gegenwart von 0,0125 Mol $FeCl_3$ (Beispiel 35) 4 Stunden auf 150°C erhitzt. Danach wurde nach destillativer Aufarbeitung die Zusammensetzung des Rohdestillats gaschromatographisch bestimmt. Die Details sind in Tabelle 6 angegeben.

T a b e l l e 6:

| Beispiel Nr. | Gehalt nach 4 Stunden b = Monofluorverbindung c = Difluorverbindung [Gew.-%] |
|---|---|
| 34+) | b 42 c 58 |
| 35 | b 24 c 75 |

+) zum Vergleich

## Patentansprüche

1. Verfahren zur Herstellung von aromatischen, kernfluorierten Verbindungen durch nucleophilen Austausch mit Kaliumfluorid in Gegenwart von Phasentransferkatalysatoren, weiteren Metallsalzen und gegebenenfalls Lösungsmitteln, dadurch gekennzeichnet, daß man die Umsetzung in zusätzlicher Gegenwart von Salzen von Metallen der 3. bis 5. Hauptgruppe des Periodensystems der Elemente und/oder von Nebengruppenelementen durchführt.

2. Verfahren nach Anspruch 1, dadurch gekennzeichnet, daß man bezogen auf ein Mol eingesetzten Phasentransferkatalysator 0,1 bis 10 Mole weitere Metallsalze einsetzt.

3. Verfahren nach Ansprüchen 1 bis 2, dadurch gekennzeichnet, daß man als Phasentransferkatalysatoren quartäre Stickstoffverbindungen, quartäre Phosphorverbindungen, Pyridiniumsalze oder Kronenether einsetzt.

4. Verfahren nach Ansprüchen 1 bis 3, dadurch gekennzeichnet, daß man Phasentransferkatalystoren in Mengen von 0,1 bis 20 Mol-°%, bezogen auf das Edukt einsetzt.

5. Verfahren nach Ansprüchen 1 bis 4, dadurch gekennzeichnet, daß man als Lösungsmittel Dimethylsulfoxid, N-Methylpyrrolidon, Tetramethylensulfon, Benzonitril, Nitrobenzol, Dimethylacetamid, Ethylenglykoldimethylether oder Diglyme einsetzt.

6. Verfahren nach Ansprüchen 1 bis 5, dadurch gekennzeichnet, daß man es im Bereich 100 bis 250° C durchführt.

7. Verfahren nach Ansprüchen 1 bis 6, dadurch gekennzeichnet, daß man pro Mol einzuführender Fluoratome 1,1 bis 2 Mole Kaliumfluorid einsetzt.

8. Verfahren nach Ansprüchen 1 bis 7, dadurch gekennzeichnet, daß man als Ausgangsmaterial 3,4-Dichlornitrobenzol, 2,4-Dichlor-nitrobenzol, 2,4-Dichlor-5-fluor-benzoylchlorid, 3,4-Dichlor-benzoylchlorid, 2,6-Dichlor-benzonitril, 2-Nitro-6-chlorbenzonitril, 2-Trifluormethyl-4-nitro-chlorbenzol, Pentachlorbenzotrifluorid, 4-Trifluormethyltetrachlor-benzoylchlorid oder 3,4,5-Trichlor-benzotrifluorid einsetzt.

9. Verfahren nach Ansprüchen 1 bis 8, dadurch gekennzeichnet, daß man alle Komponenten für das Reaktionsgemisch in trockener Form einsetzt oder im Reaktionsgemisch vorliegende geringe Wassermengen durch Abdestillieren entfernt.

## Claims

1. Process for the preparation of aromatic ring-fluorinated compounds by nucleophilic exchange with potassium fluoride in the presence of phase transfer catalysts, further metal salts and, if desired, solvents, characterized in that the reaction is carried out in the additional presence of salts of metals of main groups 3 to 5 of the periodic table of the elements and/or of sub-group elements.

2. Process according to Claim 1, characterized in that 0.1 to 10 moles of metal salt are employed relative to 1 mole of phase transfer catalyst employed.

3. Process according to Claims 1 and 2, characterized in that quaternary nitrogen compounds, quaternary phosphorus compounds, pyridinium salts or crown ethers are employed as phase transfer catalysts.

4. Process according to Claims 1 to 3, characterized in that phase transfer catalysts are employed in amounts from 0.1 to 20 mol-%, relative to the educt.

5. Process according to Claims 1 to 4, characterized in that dimethyl sulphoxide, N-methylpyrrolidone, tetramethylene sulphone, benzonitrile, nitrobenzene, dimethylacetamide, ethylene glycol dimethyl ether or diglyme are employed as solvents.

6. Process according to Claims 1 to 5, characterized in that it is carried out in the range 100 to 250°C.

7. Process according to Claims 1 to 6, characterized in that 1.1 to 2 moles of potassium fluoride are employed per mole of fluorine atoms to be introduced.

8. Process according to Claims 1 to 7, characterized in that 3,4-dichloro-nitrobenzene,2,4-dichloro-nitrobenzene, 2,4-dichloro-5-fluoro-benzoyl chloride, 3,4-dichloro-benzoyl chloride, 2,6-dichloro-benzonitrile, 2-nitro-6-chlorobenzonitrile,2-trifluoromethyl-4-nitro-chlorobenzene, pentachlorobenzotrifluoride, 4-trifluoromethyltetrachlorobenzoyl chloride or 3,4,5-trichloro-benzotrifluoride are employed as starting material.

9. Process according to Claims 1 to 8, characterized in that all components of the reaction mixture are employed in dry form or small amounts of water present in the reaction mixture are removed by distilling off.

## Revendications

1. Procédé pour fabriquer des composés aromatiques à noyau fluoré par échange nucléophile avec du fluorure de potassium en présence de catalyseurs de transfert de phase, d'autres sels métalliques et éventuellement de solvants, caractérisé en ce que l'on exécute de plus la réaction en présence de sels de métaux des groupes principaux 3 à 5 du système de classification périodique des éléments et/ou d'éléments de groupes secondaires.

2. Procédé selon la revendication 1, caractérisé en ce que l'on utilise, par mole de catalyseur de transfert de phase utilisé, 0,1 à 10 moles d'autres sels métalliques.

3. Procédé selon les revendications 1 à 2, caractérisé en ce que l'on emploie comme catalyseurs de transfert de phase, des composés d'azote quaternaires, des composés phosphorés quaternaires, des sels de pyridinium ou des éthers de couronne.

4. Procédé selon les revendications 1 à 3, caractérisé en ce que l'on emploie des catalyseurs de transfert de phase à raison de 0,1 à 20 mol-% par rapport à l'éduit.

5. Procédé selon les revendications 1 à 4, caractérisé en ce que l'on emploie comme solvant du diméthylsulfoxyde, de la N-méthylpyrrolidone, de la tétraméthylènesulfone, du benzonitrile, du nitrobenzène, du

diméthylacétamide, de l'éther diméthylique d'éthylèneglycol ou des diglymes.

**6.** Procédé selon les revendications 1 à 5, caractérisé en ce qu'on l'exécute dans une gamme de températures de 100 à 250°C.

**7.** Procédé selon les revendications 1 à 6, caractérisé en ce que l'on utilise 1,1 à 2 moles de fluorure de potassium par mole d'atomes de fluor à fixer.

**8.** Procédé selon les revendication 1 à 7, caractérisé en ce qu'on emploie comme substance de départ le 3,4-dichloronitrobenzène, le 2,4-dichloronitrobenzène, le chlorure de 2,4-dichloro-5-fluoro-benzoyle, le chlorure de 3,4-dichlorobenzoyle, le 2,6-dichlorobenzonitrile, le 2-nitro-6-chlorobenzonitrile, le 2-trifluorométhyl-4-nitrochlorobenzène, le pentachlorobenzotrifluorure, le chlorure 4-trifluorométhyltétrachlorobenzoyle ou le 3,4,5-trichlorobenzotrifluorure.

**9.** Procédé selon l'une des revendications 1 à 8, caractérisé en ce que l'on met en oeuvre tous les composants du mélange réactionnel sous forme sèche ou qu'on élimine par distillation les petites quantités d'eau présentes dans le mélanges réactionnel.